# EUROPEAN PATENT SPECIFICATION

(11) **EP 4 068 295 B1**
(45) Date of publication and mention of the grant of the patent: **30.04.2025**
(21) Application number: 21165619.4
(22) Date of filing: 29.03.2021
(51) Int. Cl.: G16H 20/10, G16H 50/50

(54) **CLINICAL DECISION SUPPORT SYSTEM FOR ESTIMATING DRUG-RELATED TREATMENT OPTIMIZATION CONCERNING INFLAMMATORY DISEASES**
KLINISCHES ENTSCHEIDUNGSUNTERSTÜTZUNGSSYSTEM ZUR ABSCHÄTZUNG DER MEDIKAMENTÖSEN BEHANDLUNGSOPTIMIERUNG IM ZUSAMMENHANG MIT ENTZÜNDLICHEN ERKRANKUNGEN
SYSTÈME DE SUPPORT DE DÉCISION CLINIQUE D'ESTIMATION DE L'OPTIMISATION DE TRAITEMENTS LIÉS AUX MÉDICAMENTS CONCERNANT DES MALADIES INFLAMMATOIRES

(43) Date of publication of application: 05.10.2022
(73) Proprietor: Siemens Healthineers AG, 91301 Forchheim (DE)
(72) Inventor: Hanke, Melanie, 90768 Fürth (DE); Jakubcík, Ján, 01001 ilina (SK); Schaller, Volker, 91052 Erlangen (DE); Vodencarevic, Asmir, 90763 Fürth (DE); Wichmann, Andre, 91054 Buckenhof (DE); Zigo, Peter, 01004 Zilina (SK); Zimmermann-Rittereiser, Marcus, 91091 Grossenseebach (DE)
(74) Representative: Siemens Healthineers Patent Attorneys

(56) References cited:
- WO-A1-2021/053343
- US-A1- 2020 321 096
- ANONYMOUS: "Feature selection - Wikipedia", 17 March 2021 (2021-03-17), XP055828372, Retrieved from the Internet <URL:https://en.wikipedia.org/w/index.php?title=Feature_selection&oldid=1012657124> [retrieved on 20210728]
- VODENCAREVIC ASMIR ET AL: "Advanced machine learning for predicting individual risk of flares in rheumatoid arthritis patients tapering biologic drugs", ARTHRITIS RESEARCH THERAPY, vol. 23, no. 1, 27 February 2021 (2021-02-27), GB, XP093134422, ISSN: 1478-6362, Retrieved from the Internet <URL:https://link.springer.com/content/pdf/10.1186/s13075-021-02439-5.pdf> [retrieved on 20240223], DOI: 10.1186/s13075-021-02439-5

## Description

The invention describes a clinical decision support ("CDS") system for estimating drug-related treatment optimization concerning inflammatory diseases, as well as a prediction-method of computed decision support and a method for manufacturing such CDS system.

Inflammatory diseases, such as rheumatic diseases like rheumatoid arthritis, psoriasis arthritis, other musculoskeletal diseases, Chronic Obstructive Pulmonary Disease (COPD), asthma, multiple skelerosis or Crohn's disease, include a wide range of medical conditions, causing chronic pain and inflammation. For example, rheumatic diseases affect joints, tendons, ligaments, muscles and bones. The most of these conditions occur when the immune system starts attacking its own tissue for still unclear reasons. Often inflammatory diseases are characterized by interlaced periods of disease inactivity, also called "remission", low and moderate disease activity as well as periods of exacerbated (high) disease activity, known as "flares".

While the most of such diseases cannot be cured, there are different types of medications which can help to keep the disease activity at low levels. Appropriate medication and dosage are e.g. specified in disease-specific guidelines (such as the ACR and the EULAR guidelines for rheumatoid arthritis). However, they are derived based on clinical studies and statistical analysis on cohort level.

Due to large differences in patients' characteristics such as demographics, diet, and lifestyle, genetic predispositions, susceptibility to external factors such as weather conditions, and likely other factors as well, it remains challenging for rheumatologists to find the right medication and/or the right dosage for an individual patient.

Often, an effective treatment needs to be changed to accommodate patient's current situation (e.g. pausing immunosuppressive therapy due to planned surgery or acute infections), lower the risk of adverse events of medication and/or reduce healthcare costs.

Furthermore, according to the "treat-to-target" strategy described in the guidelines for treating rheumatoid arthritis, the dosage of drugs and especially biologic drugs should be tapered once the stable remission is achieved. The rheumatologist is then again faced with the challenge, which patients are eligible for tapering and how much can the drug be tapered in each individual case. This is often a trial-and-error process, accompanied by reduced patient quality of life and increased healthcare costs until the correct treatment is found.

In the ambulatory clinical routine of rheumatic patients, they are examined in regular or irregular (e.g. in the case of complications like flares) time intervals by rheumatologists. During a typical patient visit, examination data is collected and sometimes previously collected demographic and lifestyle data is confirmed or updated. Based on this data, the rheumatologist makes a treatment decision ideally together with the patient. During patient's visit, a blood sample is typically taken and sent to a laboratory for analysis, most often focusing on inflammation biomarkers, such as C-reactive protein (CRP). The results of this blood test become available later, normally several days after patient's visit and after the treatment decision has already been made.

In the light of newly available lab data, the rheumatologist sometimes gains new insights and adjusts patient's treatment per phone. In all cases, treatment decisions are based on multiple relevant variables relating to patient's demographics (e.g. age, gender), examination data (e.g. patient questionnaires, numbers of tender and swollen joints), blood values (e.g. ESR, CRP), and medications (e.g. substance, co-therapy, dosage etc.).

Finally, clinical guidelines emphasize shared decision making between clinicians and patients regarding the treatment, which is not an easy task to accomplish given high number of involved relevant variables.

Specific problems are:
1. Physicians (e.g. rheumatologists) often struggle to find the initial medication and/or dosage which is likely to work for an individual patient.
2. Physicians often need to taper the drug dosage, not knowing if and how much tapering is safe and still effective for an individual patient.
3. Data relevant for treatment decisions becomes available at different time points.
4. Due to the complex nature of high-dimensional decision making in the field of inflammatory diseases (e.g. in rheumatology), data-driven black-box decision support systems are often conceived lacking transparency, which negatively affects their acceptance both by clinicians and patients.

US 2020/321096 describes computerized systems using medication-specific mathematical models for patient-specific dosing, but does not mention determination of flares.The article by Vodencarevic et al, "Advanced machine learning for predicting individual risk of flares in rheumatoid arthritis patients tapering biologic drugs", in ARTHRITIS RESEARCH THERAPY, vol. 23, no. 1, addresses discloses a clinical decision support system for estimating drug-related treatment optimization concerning inflammatory diseases and designed to output a probability of a flare connected to the application and/ or a dosage of a predefined medication, the support system having different trained prediction models to determine a risk of flares for different drug tapering scenarios and wherein the best model is selected.

So it is the object of the present invention to improve the known methods and provide a clinical decision support system for estimating drug-related treatment optimization concerning inflammatory diseases, especially a data-driven clinical decision support for therapy planning in rheumatic disease.

This object is achieved by the clinical decision support system according to claim 1, the prediction-method according to claim 10, the method for manufacturing a CDS system according to claim 11 and a data processing system according to claim 13.

In the following, the invention may be described using examples with respect to predicting the probability of flares of rheumatoid arthritis, but the invention is not limited to this application. The invention and is aspects can be used in particular for predicting the future status of a patient having a known inflammatory disease, like e.g. psoriasis arthritis, other musculoskeletal diseases, Chronic Obstructive Pulmonary Disease (COPD), asthma, multiple skelerosis or Crohn's disease.

A clinical decision support system according to the invention is defined in claim 1, while other advantageous embodiments are described in the dependent claims

In general, clinical decision support (CDS) systems are known in the art. However, this CDS system provides an estimation of a drug-related treatment optimization risk probability within a future time period. The expression "drug-related" in this context may mean in relation to a drug response and/or side effects concerning drug dosage and/or type of drug. Alternatively, "drug-related" may mean the (negative or positive) reaction of a patient on an applied drug (of a certain dose). For example, for a certain amount or type of a drug the drug response probability may be estimated (whether a drug helps or not) and/or the risk of side effects for a certain patient may be predicted.

A suitable computing unit should have enough memory and computing power to host the plurality of prediction models. This means that the computing unit must be able to process these prediction models in order to get results from input data from the prediction models. However, prediction models not used do not necessarily need to be hosted by the computing unit. They could e.g. be present in a memory until needed. Such computing units with an input interface and an output interface are known in the art. The output interface may be a data interface or a display. Any means for outputting results are possible as long as they are able to provide the data format desired by the user.

Concerning the prediction models, these are models trained for different purposes. Preferably they have been trained with training data of different groups of patients (each of these models with a training dataset concerning a different group of patients) and/or with training data relating to different medications (although a model may also be trained with a group of different drugs). The models may also be trained with different types of data (e.g. demographic data, medication data, examination data, or lab data), e.g. one model is trained with laboratory data and one with demographic data. In general, the training of prediction models as well as the architecture of these prediction models are well known in the art (see e.g. EP 3 573 068 A1).

The prediction models all have in common that each model is trained to predict the probability of treatment outcomes based on input data. These treatment outcomes may concern the response of a patient to a drug in a positive way (relief of pain, improvement of the condition), a negative way (side effects) or a neutral way (no response at all). Thus, "treatment outcomes" may be read as side effects and/or disease outcomes. This is done for a number (one or more) of different drug-related treatment options, e.g. different doses of a certain group of drugs (or a number of such groups) or different drugs. Additionally, this is done for a specific patient-group. Preferably, there are in total more than 10 different trained models used, especially more than 30 or even more than 50.

The models may be present in the computing unit itself or in a memory used by the computing unit. For example, information about the architecture and parameters of the prediction models are present in a memory and a chosen prediction model is downloaded from the memory into (a random access memory of) the computing unit.

The selection unit is designed for automatically selecting one of these prediction models. The selection is based on a predefined selection scheme and on data inputted in the CDS system to be processed by the models. The selection scheme may be a table stored in a memory of the computing system or a decision tree hardwired in the algorithm of the selection unit. Depending on the inputted data (e.g. diagnosis, lab data, data comprising information about certain drugs applied to a patient, age or gender of the patient), a model of the plurality of models is selected by the selection unit based on the selection scheme. Such selection is beneficial because it is very complicated (or even impossible) to train one single prediction model for all possible cases and for all possible patients. Furthermore, sometimes it turns out that for a special case (a special group of patients, a special disease or a special use case), a prediction model of a different architecture is better than a model with another architecture. Thus, the invention can evaluate, which prediction model (architecture and training) would be optimal for what case while constructing the CDS system and this prediction model is chosen to be part of the CDS system. Then, if the special case occurs, this model is selected by the selection unit and provides the best results for the special case.

The selection unit is preferably designed to search the input data for predefined data types and/or for values of predefined data types and to determine whether a predefined data type is present in the input data and/or to compare a value with a predefined threshold and/or to decide if the value fits a predefined requirement. For example, the selection unit may be designed to look, whether the gender of a patient is male, female or undefined, or to look whether there is lab-data available in the input data, or if diagnosis is rheumatoid arthritis for example.

The CDS system is designed to produce output results by processing the input data with the selected prediction model. How to process data with a trained model is known in the art.

Thus, depending on the used models and the selection scheme, the CDS system is able to predict response of a patient to a certain drug (concerning side effects and/or ease), or predict worsening during therapy de-escalation (especially "flare" prediction in PsA and RA and "exacerbation" prediction in COPD and Asthma). Regarding COPD, there is e.g. no de-escalation of biologics since they are to date not approved for treating these diseases but there is de-escalation of other applicable drugs, especially corticosteroids.

For example, if a patient suffering from rheumatoid arthritis is entering phase 1 of a treatment, the CDS system can estimate, whether this patient will positively respond to drugs that are suggested by corresponding guidelines, approved for use and available at the treating institution. If the model will predict that this special patient will not respond to any phase 1 drugs, then phase 2 could be started immediately, sparing months of suffering due to non-effective drugs or side effects.

A prediction-method according to the invention of computed decision support comprises the following steps:
- providing a CDS system according to the invention,
- providing input data to the CDS system, wherein the input data is preferably selected and provided automatically, especially if new data becomes available for a predefined patient,
- determining a result with the CDS system wherein a prediction model is selected automatically by the CDS system based on the input data and the result is determined automatically by the selected prediction model,
- outputting the result, especially wherein a user is notified if substantial changes in a result for a patient occurred compared to earlier results for the same patient, e.g., in the form of a warning message or an icon in the patient list, so that he knows that he has to open the case.

The input data is preferably data relating to one single patient and preferably comprises data from the group of demographic data (possibly including lifestyle data), medication data, examination data and lab data. Additionally, the input data comprises information about an intended change of treatment, e.g. information about a drug intended to be applied to the patient or a reduction or increase of a drug dose. Moreover, the input data could potentially include omics data (e.g. proteomics, genomics, metabolomics) and medical image data acquired using different imaging modalities (e.g. magnetic resonance imaging, ultrasound, computed tomography).

A method according to the invention for manufacturing a CDS system according to the invention comprises the following steps:
- providing at least a first model-group and a second model-group, each model-group having a plurality of untrained machine learning models, especially with a number of models having a different internal architecture and/or different hyperparameters,
- providing at least a first training-dataset and a second training-dataset, each training-dataset comprising data with a different distinguishing feature,
- training of the first model-group with the first training-dataset and the second model-group with the second training-dataset,
- ranking each trained prediction model of a model-group with a predefined quality-criterion, preferably wherein prediction models are developed and compared offline,
- choosing the best ranked prediction model of each model-group as prediction model for the clinical decision support system manually or automatically.

It should be noted that the model-groups mentioned here are not the models of the CDS system. Only the "winner" of a group (or winners) will take a place in the final CDS system. In praxis, there should be more groups than one, e.g. more than 10, more than 30 or more than 50.

The training datasets should be chosen on behalf of the purpose of the individual trained model. If a model is to be applied for patients of the age of 60 or older, the training dataset should only comprise data of patients of the age 60 or older. If the model is to be applied for a certain drug, the respective training dataset should comprise data about patient response based on this drug.

The training criterion could depend on the state of the patients the training data were taken from. The criterion could allocate a quality score to a prediction model in the case a validation occurs and the prediction quality of the trained models is quantized. The training criterion could also be derived from the criterions of a nested cross validation process.

For example, there is a number of predictive models (e.g. 52) trained for different diseases, medications, actions (application of new drugs or drug-reduction) and patient-groups. These models could then be used in the CDS system for, e.g. RA (rheumatoid arthritis); Phase II (EULAR guidelines); prediction of response to a certain drug, or PsA (psoriasis arthritis); Phase IV, tapering (also known as "dose reduction" or "therapy de-escalation"). The best model is automatically chosen for the given purpose by the selection unit.

Regarding a patient with a certain disease, the selection can be accomplished by determining which of the predictive models is trained with a group of patients that has the most similarities with the actual patient, respectively which of the predictive models is trained with patients having the actual disease. Regarding information about a (planned or applied) medication in the input data, it may be determined, which of the predictive models is trained with a group of patients getting these drugs. Regarding certain types of input data (e.g. lab data), it may be determined, which of the predictive models is trained with such data.

A data processing system of the invention, that is especially a computer network system, comprises a data-network, a number of client computers and a service computer-system, wherein the service computer system comprises a Clinical Decision Support System according to the invention.

The units or modules of the invention mentioned above can be completely or partially realised as software modules running on a processor of a computing system. A realisation largely in the form of software modules can have the advantage that applications already installed on an existing system can be updated, with relatively little effort, to install and run the methods of the present application. The object of the invention is also achieved by a computer program product with a computer program that is directly loadable into the memory of a computing system and which comprises program units to perform the steps of the inventive method when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

Regarding the trained prediction models or their training, there are some parameters (or "data") that refer to different types of data. There is preferably demographic data, medication data, examination data or laboratory data (also including related scores and derived variables).

Demographic data may be data referring to patient, especially gender (male, female), height, weight, body mass index, age, smoking status (never smoked, yes, ex), alcohol intake (yes, no, amount), list of comorbidities, time since a diagnosis has been made.

Preferred medication data is data referring to a treatment with an active agent, preferably as listed below, especially biologics/biosimilars, methotrexate, other conventional disease-modifying antirheumatic drugs (cDMARDs), targeted synthetic disease-modifying antirheumatic drugs (tsDMARTs), nonsteroidal anti-inflammatory drugs (NSAID), glucocorticoids. Referring to any of the active agents, the data may refer to any member of the group treatment (yes/no), actual substance, administration way, prescribed dosage, prescribed interval, start time and stop time.

Preferred examination data is data referring to a tender joint count (e.g. 0 to 28), swollen joint count (e.g. 0 to 28), patient assessment of pain (e.g. 0 to 100), patient assessment of disease activity (e.g. 0 to 100), doctor assessment of disease activity (e.g. 0 to 100), health Assessment Questionnaire (e.g. 0 to 3), "Funktionsfragebogen Hannover" (e.g. 0 to 100), clinical disease activity index (e.g. 0 to 76).

Preferred lab data is data referring to the rheumatoid factor (positive, negative), Anti-Cyclic Citrullinated Peptides (positive, negative), seropositive rheumatoid arthritis (positive, negative), C-Reactive protein (e.g. >0.01), erythrocyte sedimentation rate (e.g. 0 to 100), Disease Activity Score based on ESR (e.g. 0 to 9.1), Disease Activity Score based on CRP (e.g. 0 to 8), simple disease activity index (e.g. 0 to 86), duration of remission (e.g. >=0), count of previous flares (e.g. >=0).

All these possible data could be included in the input data and be used by the selection unit.

According to a preferred embodiment of the CDS system, for a number of the different prediction models, each prediction model has been trained for a different patient-group and is selected based on patient-relating information in the input data, preferably based on demographic data and/or on examination data, especially based on information concerning distinguishing features from the group comprising gender, type of disease (e.g. seropositive vs. seronegative), age, underlying health condition, body mass index. This means that there are prediction models that are specially trained for special groups of patients that can be recognized by special values of patient related data. For different patients with different respective values, different prediction models are automatically chosen.

According to a preferred embodiment of the CDS system, for a number of the different prediction models, each prediction model has been trained for a different location in a clinical pathway and is selected based on input data referring to the patient's location in a clinical pathway, preferably based on examination data.

According to a preferred embodiment of the CDS system, for a number of the different prediction models, each prediction model has been trained for a different medication and is selected based on a type of medication given (indicated) in the input data, the medication especially based on cDMARDs (lightweight cheaper conventional disease modifying antirheumatic drugs), e.g. on methotrexate, sulfasalazine, hydroxychloroquine and leflunomide. There are however many biologic DMARDs (expensive, partially severe side effects but typically much higher effect on the disease activity). More recently, there are also biosimilars and targeted synthetic DMARDs on the market. And there are also NSAID (e.g. aspirin, ibuprofen) for very light symptoms and also dangerous steroidal drugs like glucocorticoids or cortisone for short-term application in cases of acute severe flares.

Suitable active agents (medication) for special inflammatory diseases are listed below:
Preferred drugs used in the treatment of rheumatoid arthritis are conventional disease-modifying antirheumatic drugs (cDMARD) or biologics or other drugs that temporarily ease pain and inflammation. Preferred cDMARDs used to treat RA include hydroxychloroquine, leflunomide, methotrexate, sulfasalazine or minocycline. Preferred biologics include abatacept, rituximab, tocilizumab, anakinra, adalimumab, etanercept, infliximab, certolizumab pegol or golimumab. Preferred tsDMARDs include Janus associated kinase inhibitors like tofacitinib or baricitinib. Preferred nonsteroidal anti-inflammatory drugs (NSAIDs) comprise ibuprofen /hydrocodone, ibuprofen/oxycodone, naproxen sodium, aspirin, celecoxib, nabumetone, naproxen (-sodium), piroxicam, diclofenac, diflunisal, indomethacin, ketoprofen, etodolac, fenoprofen, flurbiprofen, ketorolac, meclofenamate, mefenamic acid, meloxicam, oxaprozin, sulindac, salsalate, tolmetin, diclofenac/misoprostol, topical capsaicin or opioid pain drugs like codeine, acetaminophen/codeine, fentanyl, hydrocodone, hydromorphone, morphine, meperidine, oxycodone, tramadol. Preferred steroidal drags include corticosteroids like beta-methasone, prednisone, dexamethasone, cortisone, hydrocortisone, methylprednisolone, prednisolone. Preferred immunosuppressants comprise cyclosporine, cyclophosphamide, azathioprine or hydroxychloroquine.

Preferred drugs used in the treatment of psoriatic arthritis (PsA) include disease-modifying anti-rheumatic drugs (DMARD) immunosuppressants, and tumor necrosis factor-alpha (TNF-alpha) inhibitors. Preferred DMARDs used to treat PsA include methotrexate, sulfasalazine, cyclosporine or leflunomide.

Preferred nonsteroidal anti-inflammatory drugs (NSAIDs) comprise ibuprofen or naproxen. A preferred immunosuppressant drug comprises azathioprine. Preferred TNF-alpha inhibitors comprise adalimumab, etanercept, golimumab or infliximab.

Preferred drugs used in the treatment of Chronic Obstructive Pulmonary Disease (COPD) are for example short-acting bronchodilators, corticosteroids, methylxanthines, long-acting bronchodilators, combination drugs, roflumilast, mucoactive drugs, vaccines, antibiotics, cancer medications or biologic drugs. Examples of short-acting bronchodilators include albuterol, levalbuterol, ipratropium or albuterol /ipratropium. Preferred corticosteroids include fluticasone or prednisolone. Preferred long-acting bronchodilators are aclidinium, arformoterol, formoterol, glycopyrrolate, indacaterol, olodaterol, revefenacin, salmeterol, tiotropium or umeclidinium. Recommended LABA/LAMA combination bronchodilator therapies include aclidinium/formoterol, glycopyrrolate /formoterol, tiotropium/olodaterol or umeclidinium/vilanterol. Combinations of an inhaled corticosteroid and a long-acting bronchodilator include budesonide/formoterol, fluticasone/salmeterol or fluticasone/vilanterol.

Preferred drugs used in the treatment of asthma are bronchodilators or anti-inflammatories, respecively quick-relief medications or long-term asthma control medications. Preferred are short-acting beta agonists like albuterol or levalbuterol. Preferred are also anticholinergic like ipratropium bromide (Atrovent HFA). Preferred long-term asthma control medications comprise inhalable corticosteroids like beclomethasone, budesonide, flunisolide, fluticasone or mometasone; corticosteroids like prednisone, methylprednisolone or hydrocortisone; long-acting beta agonists like formoterol or salmeterol. Preferred combination inhalers comprise budesonide and formoterol or fluticasone and salmeterol. Preferred leukotriene modifiers comprise montelukast, zafirlukast or zileuton. Preferred methylxanthines comprise theophylline. Preferred immunomodulators comprise mepolizumab, omalizumab or reslizumab.

Preferred drugs used in the treatment of multiple skelerosois (MS) are interferon beta-1b, interferon beta-1a, glatiramer acetate, peginterferon beta 1-a, mitoxantrone, natalizumab, fingolimod, or other sphingosine-1-phosphate receptor modulators, teriflunomide, pyrimidine, cladribine, ocrelizumab, siponimod, cladribine, diroximel fumarate, ozanimod, monomethyl fumarate.

Preferred drugs used in the treatment of Crohn's disease are medications to treat any infection (normally antibiotics) and to reduce inflammation (normally aminosalicylate anti-inflammatory drugs and corticosteroids). Medications used to treat the symptoms of Crohn's disease especially include 5-amino-salicylic acid (5-ASA) formulations, prednisone, immunomodulators such as azathioprine (given as the prodrug for 6-mer-captopurine), methotrexate, infliximab, adalimumab, certolizumab, vedolizumab, ustekinumab and natalizumab. Hydrocortisone should be used in severe attacks of Crohn's disease.

Some of the above mentioned active agents, e.g. the TNF-alpha inhibitors or immunomodulators belong to the group of expensive biologic drugs.

A preferred embodiment of the CDS system is designed to select a prediction model based on the types of input data available, preferably wherein a prediction model is selected depending on the case whether or not lab data is part of the input data. This has the advantage that in the case of a preliminary talk or examination (without lab results) a prediction model can be chosen that allows a first impression of possible results and after lab data is available, another prediction model is automatically chosen that allows an enhanced and optimized prediction. The availability of distinct data items may be independent from the pathway (also for a non-treatment-naïve patient there may be no recent lab data available). A selection based on the different kinds of data available is advantageous. If lab data is not available other models should be used than with lab data available.

Since there are many possible constellations of input data and inflammatory diseases, in the following there are listed some explaining examples referring rheumatoid arthritis.

There are three phases listed in the EULAR guidelines for treatment of RA with pharmacological non-topical treatments, cDMARDs, biological disease-modifying antirheumatic drugs (bDMARDs) and targeted synthetic disease-modifying antirheumatic drugs (tsDMARDs).

In the first phase, there is often made a selection between methotrexate and sulfasalazine combined with short term glucocorticoids. The CDS system could be used to predict the effectiveness of these drugs and provide help for the estimation of the applied drug. The input data for the prediction could be demographic data and examination data of the respective patient. Predictive models could be trained on said drugs and the response of patients concerning these drugs.

However, in this phase also a predictive model could be selected if a tapering of an already applied drug is planned (i.e. if patient is in sustained remission). Then, the input data for the prediction could comprise demographic data and examination data of the respective patient together with information about the applied drug. Predictive models could be trained on dose reduction in sustained remission scenarios of the respective drug and the response of patients to tapering.

In the second phase, expensive active agents are typically applied. Often, a selection is made between the addition of a bDMARD or a JAK-inhibitor on the one hand and the change of the already applied cDMARD or an addition of a cDMARD on the other hand. The CDS system could be used to predict the effectiveness of these alternatives and provide help for the selection. The input data for the prediction could again be demographic data and examination data of the respective patient in addition to data about the applied drugs. Predictive models could be trained on said data and the response of patients concerning the respective drugs.

However, in this phase also a predictive model could be selected in the case dose reduction or an interval increase is planned in sustained remission. Then also input data for the prediction could be demographic data and examination data of the respective patient together with information about the applied drugs. Predictive models could be trained on dose reduction or interval increase in sustained remission scenarios of the respective drugs and the response of patients.

In the third phase, there is often made a decision whether an applied medication should be changed (e.g. another bDMARD or a JAK-inhibitor). The CDS system could be used to predict the effectiveness of such change. The input data for the prediction could be demographic data and examination data of the respective patient in addition to the applied drugs. Predictive models could be trained on said drugs and the response of patients concerning these drugs.

However, as in phase 2, in this phase also a predictive model could be selected in the case dose reduction or an interval increase is planned in sustained remission. Then also input data for the prediction could be demographic data and examination data of the respective patient together with information about the applied drugs. Predictive models could be trained on dose reduction or interval increase in sustained remission scenarios of the respective drugs and the response of patients.

For example, one study showed that in phase 1, methotrexate is not effective with about 43% of the patients (see e.g. https://arthritis-research.biomedcentral.com/articles/ 10.1186/s13075-018-1645-5). Thus, it would be advantageous to identify those patients that positively respond to methotrexate and those who do not. In the course of dose reduction or interval increase, the probability of flares (within a certain time horizon) could be computed by the prediction models.

Concerning psoriasis arthritis, there is a similar EULAR guideline comprising four phases with a similar procedure as described above. Here also, effects of the application of a new drug or the risk of flares following drug-tapering could be predicted by automatically selecting a predictive model.

Preferably, a first selection of a predictive model is based on the diagnosis of a physician (type of disease and phase of EULAR guideline), and the demographic data of the patient. Then the drug(s) applied or planned to apply could be part of the input data, as well as the planned actions (response prediction or dose reduction). Last the presence of certain data types (e.g. only examination data or also lab data) may also be a criterion of selection of the used prediction model. Last, historic data (anamnesis of the patient), comorbidities or lifestyle of a patient, potentially available omics data (e.g. metabolomics, proteomics, genomics) and imaging data (e.g. computed tomography images) may also be part of the input data and basis for selection of a predictive model.

According to a preferred embodiment of the CDS system, a number of the different prediction models is trained to determine a probability that an individual patient will respond to a specific drug and/or a risk of flares for different drug tapering scenarios and/or a risk of drug adverse events.

It is preferred that a prediction model is trained for
- determining a response probability for first line drugs, e.g. methotrexate and sulfasalazine, and/or
- determining a selection of the second line drug and/or
- drug tapering in any treatment stage (according to EULAR tapering recommendations), especially for RA patients receiving biologics in stable remission, preferably for a plurality of dosage regimes.

According to a preferred embodiment of the CDS system, a number of prediction models is trained to determine drug response of a patient for a plurality of drugs, preferably wherein one single model determines drug response of a patient for a plurality of drugs, and/or a model of a group of multiple models determines drug response of a patient for one single drug.

A preferred embodiment of the CDS system is designed to output a probability of a flare (especially connected to the application and/or a dosage of a predefined medication), a probability of an adverse events (e.g. side effects of medication) and/or a probability of a patient non responding to a drug. For example, the CDS system is designed to predict the numeric value of relevant disease activity scores such as DAS28-ESR in RA patients (e.g. instead or additionally to predicting flares), which are defined as DAS28-ESR > 2.6.

Preferably prediction models of the clinical decision support system are trained to determine and output a confidence score for a prediction, preferably wherein a prediction is a binary value referring to a classification and the confidence is a probability value and/or preferably wherein the prediction is a regression the output comprises prediction intervals for point predictions.

A preferred embodiment of the CDS system is designed to output information about which input group of parameters affect the output the most, preferably designed to generate for individual parameters of this group a value of how much they affect the output information. This provides a quantitative impression of the importance of these parameters. For example, if it is evident, that for a certain patient a result strongly depends on the body mass index, there could be made specific efforts to positively change the body mass index. Thus, an advantage of this embodiment is that a user could infer from the output that a high flare risk is due to a specific medication regime and selectively change it.

According to a preferred method for manufacturing a CDS system, a prediction method according to the invention is performed with the clinical decision support system according to the invention and a feedback-dataset is provided for a number of patients, wherein the prediction models are further trained with this feedback dataset. It should be noted that the prediction models are connected to the distinguishing feature of the feedback data. Preferably, a feedback-dataset is used for training, where a patient had a flare with a DAS28_ESR score higher than 2.6.

In the preferred case of a CDS system for rheumatoid arthritis (but also applicable for other diseases), there is a plurality of prediction models (especially more than 10), that are specially trained for different input data.

There could be several groups of such prediction model, wherein each group comprises a plurality of prediction models (especially more than 10), that are specially trained for different input data for different diseases (e.g. RA, PsA, Spondyloarthropathy - SpA). The selection unit is then designed to parse input data for information about a diagnosis for a disease to determine the actual group of prediction models that should be used for selecting an individual prediction model, i.e. to filter all prediction models if they are trained with data referring to this disease.

Preferably, there is a plurality of prediction models, that are specially trained for different phases of treatment in the course of a certain disease. A preferred selection unit parses the input data regarding information about the phase of treatment and filters the prediction models accordingly (especially together with a filter regarding a certain disease) depending on their training.

As can be seen, it is preferred to label the prediction models, on what special data they are trained, e.g. could the label comprise information about a disease, a phase of treatment, patients (e.g. gender, age, BMI), medication or use cases (response to a drug or drug tapering).

Preferably, there is a plurality of prediction models, that are specially trained for different use cases (e.g. change of medication or tapering of medication). A preferred selection unit parses the input data regarding information about the use case and filters the prediction models accordingly (especially together with a filter regarding a certain disease and/or a phase of treatment) depending on their training.

Preferably, there is a plurality of prediction models, that are specially trained on lab data and other that are trained on other examination data (e.g. examination by a physician). A preferred selection unit parses the input data by looking whether there is lab/examination data available or not and filters the prediction models accordingly (especially together with a filter regarding a certain disease and/or a phase of treatment and/or a use case) depending on their training.

Wherever not already described explicitly, individual embodiments, or their individual aspects and features, can be combined or exchanged with one another without limiting or widening the scope of the described invention, whenever such a combination or exchange is meaningful and in the sense of this invention. Especially some features described here could form individual inventions, especially in combination with other features of this description. Advantages which are described with respect to one embodiment of the present invention are, wherever applicable, also advantageous of other embodiments of the present invention.

Other objects and features of the present invention will become apparent from the following detailed descriptions considered in conjunction with the accompanying drawings. It is to be understood, however, that the drawings are designed solely for the purposes of illustration and not as a definition of the limits of the invention.
- Fig. 1: displays a preferred data processing system with a CDS system according to the invention.
- Fig. 2: displays a block diagram of a prediction-method according to the invention.
- Fig. 3: displays a block diagram of a method for manufacturing a CDS system according to the invention.
- Fig. 4: displays an EULAR-scheme for the treatment of rheumatoid arthritis.
- Fig. 5: displays an EULAR-scheme for the treatment of psoriasis arthritis.
- Fig. 6: displays a possible decision tree for the selection unit.

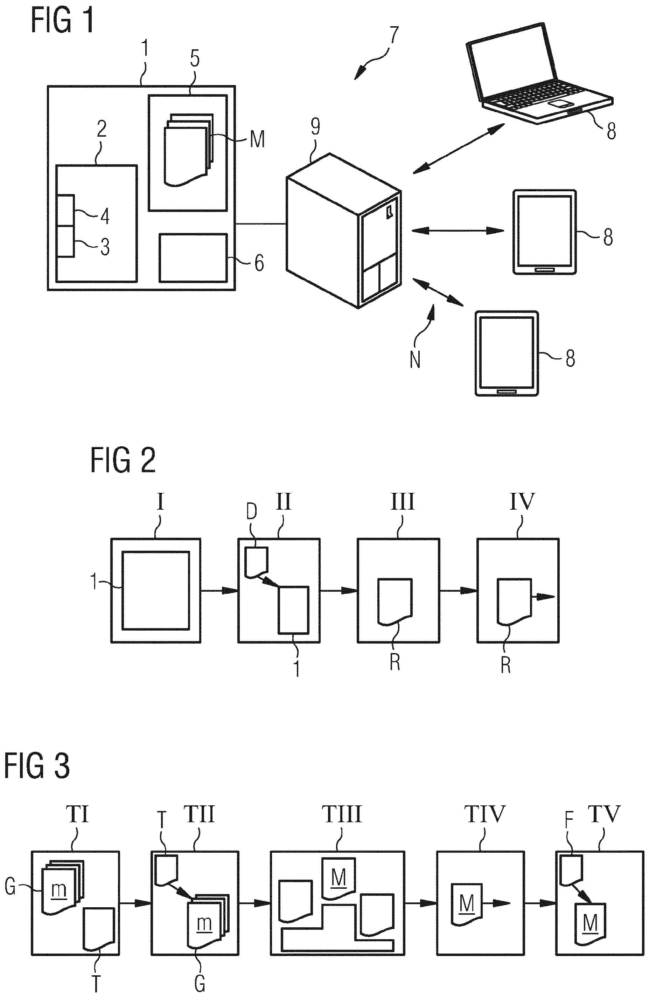

Figure 1 displays a preferred data processing system 7 with a CDS system 1 according to the invention. The data processing system 7 comprises client computers 8 connected with a service computer-system 9 via a data-network N. The service computer system 9 comprises a clinical decision support system 1 according to the invention.

The clinical decision support system 1 for estimating drug-related treatment optimization concerning inflammatory diseases, comprises the following components:
A computing unit 2 comprising an input interface 3 designed for receiving input data D (see following figures) and an output interface 4 designed to output results R. The computing unit 2 is designed to host a plurality of prediction models M, i.e. to process these prediction models M in order to get results R from input data D from the prediction models M. However, prediction models not used do not need to be actively hosted by the computing unit 2.

A memory 5 to save and provide the multiple prediction models M for the case a prediction model M is needed by the computing unit 2.

A plurality of different trained prediction models M that are here saved in said memory 5. Each model M is trained to predict the probability of treatment outcomes for a number of different drug-related treatment options and for a specific patient-group. For example, some prediction models M are trained for different patient-groups and should be selected based on patient-relating information in the input data D, some prediction models M are trained for different locations in a clinical pathway and are selected based on respective input data D, or some prediction models M are trained for different medications and are selected based on a type of medication given in the input data D.

The prediction models M are preferably trained to determine a probability that an individual patient will respond to a specific drug and/or a risk of flares for different drug tapering scenarios, especially for determining a response probability for first line drugs or determining a selection of the second line drug or for drug tapering in a later treatment stage (i.e. not the actual stage or phase). There could be prediction models M trained only for one single drug or for a plurality of drugs.

A selection unit 6 designed for automatically selecting one of these prediction models M depending on the input data D according to a predefined selection scheme. A prediction model M may e.g. be selected based on diagnosis, the types of input data D available, preferably wherein a prediction model M is selected depending on on the case whether or not lab data is part of the input data D.

The clinical decision support system 1 is designed to produce output results R by processing the input data D with the selected prediction model M. Especially, the clinical decision support system 1 is designed to output a probability of a flare, a probability of an adverse events (e.g. side effects of medication) and/or a probability of a patient non responding to a drug. To achieve this, prediction models M of the clinical decision support system 1 may be trained to determine and output a confidence score for a prediction, preferably wherein a prediction is a binary value referring to a classification and the confidence is a probability value and/or preferably wherein the prediction is a regression the output comprises prediction intervals for point predictions.

The clinical decision support system 1 may be designed to output information (e.g. in the results) about which input group of parameters affect the output the most, preferably designed to generate for individual parameters of this group the value of how much they affect the output result R. This could e.g. be achieved by using SHAP explainable AI framework (Shapley Additive exPlanations).

Figure 2 displays a block diagram of a prediction-method according to the invention.

In step I, a clinical decision support system 1 is provided, e.g. as shown in figure 1.

In step II, input data D is provided to the clinical decision support system 1, wherein the input data D may be selected and provided automatically, especially if new data becomes available for a predefined patient. However, also a physician can upload a chosen dataset into the CDS system 1.

In step III, a result R is determined with the clinical decision support system 1 wherein a prediction model M is selected automatically by the clinical decision support system 1 based on the input data D. The result R is determined automatically by the selected prediction model M.

In step IV, the result R is outputted by the CDS system 1. A user may be notified, if substantial changes in a result for a patient occurred compared to earlier results for the same patient, e.g. in the form of a warning message or an icon in the patient list, so that it is indicated to open the case.

Figure 3 displays a block diagram of a method for manufac-turing a CDS system 1 according to the invention (see e.g. fig-ure 1).

It should be noted that only one single model-group G is regarded in this example, although the method uses two or more (preferably multiple) model-groups G and a respective number of training datasets T. However, the procedure is similar for each model-group G.

In step TI, a model-group G having a plurality of untrained machine learning models m is provided. It is preferred that the untrained models m have a different internal architecture and/or different hyperparameters, so it could be evaluated, which architecture / hyperparameters would be the best for a certain task.

Also in step TI, a training dataset T is provided, comprising data with a different distinguishing feature compared to other training datasets T. For example, all patients are female or the training dataset T comprises lab data.

In step TII, training of the model-group G is performed with the training-dataset T.

In step TIII, the trained prediction models M of the model-group G are ranked with predefined quality criteria. It can be seen that a trained prediction model M is the "winner" of this ranking. The prediction models M could be developed and compared offline.

In step TIV, the best ranked prediction model M of the model-group G is chosen as prediction model M for the clinical decision support system 1 manually or automatically.

In step TV, a feedback dataset F is provided for a number of patients and the (chosen) prediction models M of the CDS system 1 are further trained with this feedback dataset F. The prediction models M are here connected to the distinguishing feature of the feedback dataset F. For example, a feedback dataset F could be used for training, where a patient had a flare with a DAS28-ESR score higher than 2.6. A flare could also be self-reported by a patient, which is also included in a feedback dataset F.

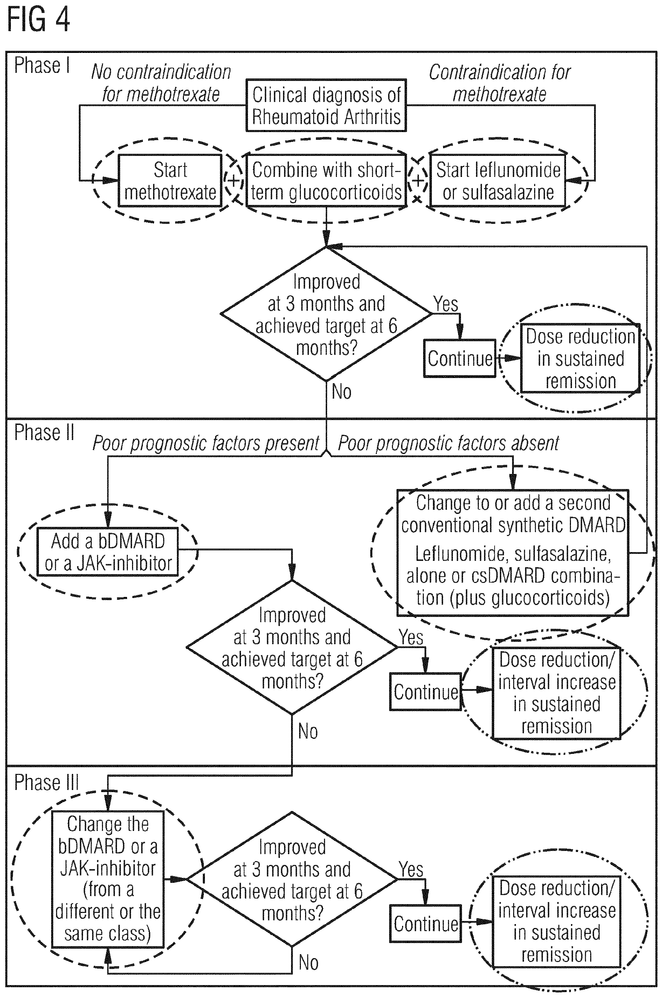

Figure 4 displays an EULAR schematic guideline for the treatment of rheumatoid arthritis. There are three phases listed in the EULAR guidelines for treatment of RA with cDMARDs possibly in combination with glucocorticoids, bDMARDs possibly in combination with cDMARDs and tsDMARDs such as JAK-inhibitors. There are added dashed ellipses for the treatment decisions that may be supported by prediction models M specially trained for the response to drugs and dash-dotted ellipses for the treatment decisions that may be supported by prediction models M specially trained for drug tapering.

In the first phase, there is often made a selection between methotrexate and sulfasalazine combined with short term glucocorticoids. The CDS system 1 could be used to predict the effectiveness of these drugs and provide help for the estimation of the success of the applied drug. The input data D for the prediction could be demographic data and examination data of the respective patient. Predictive models M could be trained on said drugs and the response of patients concerning these drugs.

However, in this phase also a predictive model M could be selected in the case tapering of an already applied drug is planned, assuming the patient is in sustained remission. Then also input data D for the prediction could be demographic data and examination data of the respective patient together with information about the applied drug. Predictive models M could be trained on dose reduction in sustained remission scenarios of the respective drug and the response of patients to tapering.

In the second phase, more expensive active agents are typically applied. There is often made a selection between the addition of a bDMARD or a JAK-inhibitor (tsDMARD) on the one hand and the change of the already applied bDMARD or an addition of a cDMARD on the other hand. The CDS system 1 could be used to predict the effectiveness of these alternatives and provide help for the selection. The input data D for the prediction could again be demographic data and examination data of the respective patient in addition to the applied drugs. Predictive models M could be trained on said drugs and the response of patients concerning these drugs.

However, in this phase also a predictive model M could be selected in the case dose reduction or an interval increase is planned in sustained remission. Then also input data D for the prediction could be demographic data and examination data of the respective patient together with information about the applied drugs. Predictive models M could be trained on dose reduction or interval increase in sustained remission scenarios of the respective drugs and the response of patients.

In the third phase, there is often made a decision whether an applied medication should be changed (e.g. another bDMARD or a JAK-inhibitor due to poor prognostic factors or ineffectiveness or adverse events observed in the second phase). The CDS system 1 could be used to predict the effectiveness of such drug change. The input data D for the prediction could be demographic data and examination data of the respective patient in addition to the applied drugs. Predictive models M could be trained on said drugs and the response of patients concerning these drugs.

However, as in phase 2, in this phase also a predictive model M could be selected in the case dose reduction or an interval increase is planned in sustained remission. Then also input data D for the prediction could be demographic data and examination data of the respective patient together with information about the applied drugs. Predictive models M could be trained on dose reduction or interval increase in sustained remission scenarios of the respective drugs and the response of patients.

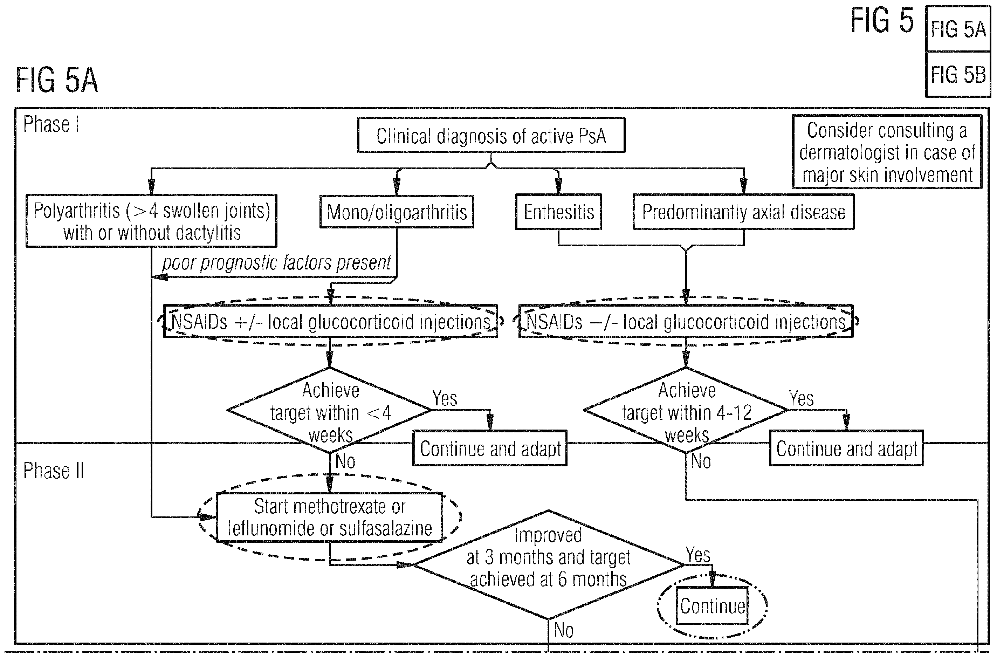

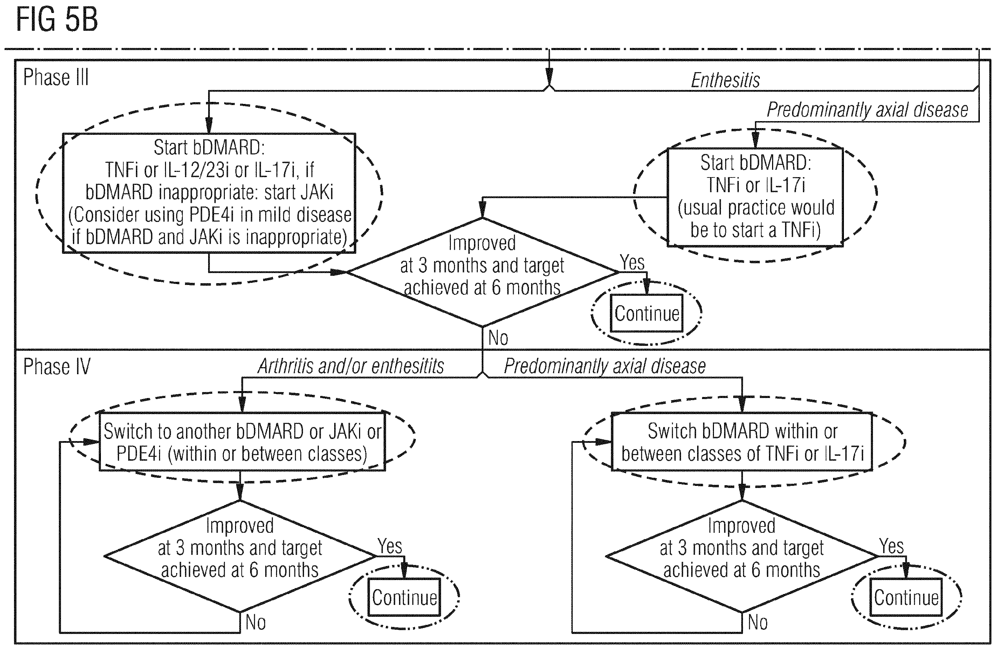

Figure 5 displays an EULAR-scheme for the treatment of psoriasis arthritis. There are four phases listed in the EULAR guidelines for treatment of PsA, wherein the algorithm is similar to the treatment of RA. Again, there are added dashed and dash-dotted ellipses for these parts that may be predicted by predicting models M specially trained for the response to drugs.

Concerning psoriasis arthritis, there is a similar EULAR guideline comprising four phases with a similar procedure as described above. Here also, effects of the application of a new drug or the risk of flares following drug tapering could be predicted by automatically selecting a predictive model M.

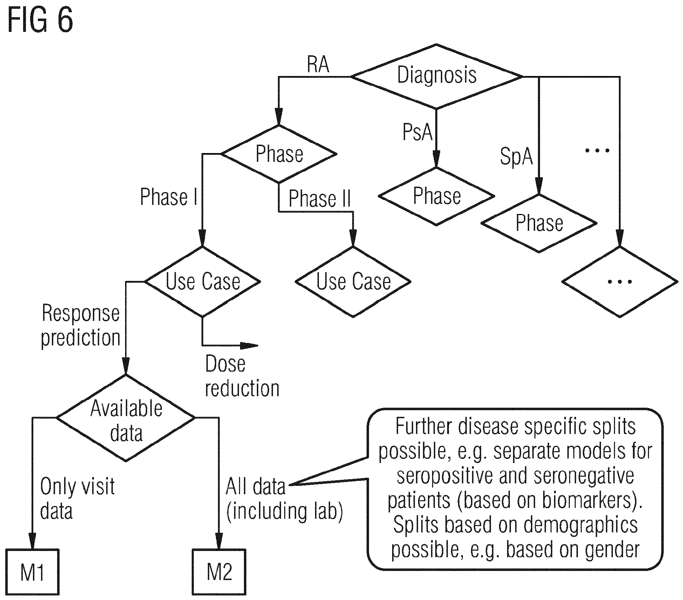

Figure 6 displays a possible decision tree for the selection unit 6 (see above figures 1 and 2).

At first (upper part), there is made a diagnosis to determine the actual disease a patient suffers from. This information is entered in the input data D and the selection unit 6 is designed to determine from the input data D the actual disease and selects prediction models M that are trained on this disease. However, there may be a vast number of possible prediction models M so that the selection should be filtered.

At second (next phase from top to bottom), the phase of treatment (see e.g. figures 4 and 5) is added to the input data D and the selection unit 6 could be designed to determine from the input data D the actual phase and select prediction models M that are trained for this phase.

Third (next phase from top to bottom), the use case (change of medication or tapering of medication) could be added to the input data D and the selection unit 6 could be designed to select from the input data D prediction models M that are trained for the prediction of the influence of certain drugs on patients or the influence of drug tapering on patients.

Next (bottom phase), it could be automatically checked by the selection unit 6, whether there is examination and/or lab data available in the input data D and select prediction models M that are trained for make predictions on such data.

Although the present invention has been disclosed in the form of preferred embodiments and variations thereon, it will be understood that numerous additional modifications and variations could be made thereto without departing from the scope of the invention, as defined by the appended claims.

For the sake of clarity, it is to be understood that the use of "a" or "an" throughout this application does not exclude a plurality, and "comprising" does not exclude other steps or elements. The mention of a "unit" or a "module" does not preclude the use of more than one unit or module.

## Claims

1. A clinical decision support system (1) for estimating drug-related treatment optimization concerning inflammatory diseases and designed to output a probability of a flare connected to the application and/or a dosage of a predefined medication, the clinical decision support system (1) comprising:
- a computing unit (2) designed to host a plurality of prediction models (M), the computing unit (2) comprising an input interface (3) designed for receiving input data (D) and an output interface (4) designed to output results (R),
- a plurality of different trained prediction models (M), wherein each model (M) is trained to predict the probability of treatment outcomes for a number of different drug-related treatment options and for a specific patient-group based on input data, and wherein a number of the different prediction models is trained to determine a risk of flares for different drug tapering scenarios,
- a selection unit (6) designed for automatically selecting one of these prediction models (M) depending on the input data (D) according to a predefined selection scheme,
wherein the clinical decision support system (1) is designed to produce output results (R) by processing the input data (D) with the selected prediction model (M),
and wherein the selection unit is designed to search the input data for predefined data types and/or for values of pre-defined data types and to determine whether a predefined data type is present in the input data and/or to compare a value with a predefined threshold and/or to decide if the value fits a pre-defined requirement.

2. The clinical decision support system according to claim 1, wherein for a number of the different prediction models (M), each prediction model (M) has been trained for a different patient-group and is selected based on patient-relating information in the input data (D), preferably based on demographic data and/or on examination data, especially based on information concerning distinguishing features from the group comprising gender, type of disease (e.g. seropositive vs. seronegative rheumatoid arthritis), age, underlying health condition, body mass index.

3. The clinical decision support system according to one of the preceding claims, wherein for a number of the different prediction models (M), each prediction model (M) has been trained for a different location in a clinical pathway and is selected based on input data (D) referring to the patient's location in a clinical pathway, preferably based on examination data.

4. The clinical decision support system according to one of the preceding claims, wherein for a number of the different prediction models (M), each prediction model (M) has been trained for a different medication and is selected based on a type of medication given in the input data (D), the medication especially based on DMARDs, e.g. bDMARDs, cDMARDs or tsDMARDs, or NSAIDs.

5. The clinical decision support system according to one of the preceding claims, designed to select a prediction model (M) based on the types of input data (D) available, preferably wherein a prediction model (M) is selected depending on the case whether or not lab data is part of the input data (D).

6. The clinical decision support system according to one of the preceding claims, wherein a number of the different prediction models (M) is trained to determine a probability that an individual patient will respond to a specific drug and/or a risk of flares for different drug tapering scenarios, preferably wherein a prediction model (M) is trained for
- determining a response probability for first line drugs, e.g. methotrexate and sulfasalazine, and/or
- determining a selection of the second line drug and/or
- a drug tapering scenario in a later treatment stage, especially for RA patients receiving biologics in stable remission, preferably for a plurality of dosage regimes.

7. The clinical decision support system according to one of the preceding claims, wherein a number of prediction models (M) is trained to determine drug response of a patient for a plurality of drugs, preferably wherein one single model determines drug response of a patient for a plurality of drugs, and/or a model of a group of multiple models determines drug response of a patient for one single drug.

8. The clinical decision support system according to one of the preceding claims, designed to output a probability of a flare, especially connected to the application and/or a dosage of a predefined medication, a probability of an adverse event (e.g. side effects of medication) and/or a probability of a patient not responding to a drug,
preferably wherein prediction models (M) of the clinical decision support system (1) are trained to determine and output a confidence score for a prediction, preferably wherein a prediction is a binary value referring to a classification and the confidence is a probability value and/or preferably wherein the prediction is a regression the output comprises prediction intervals for point predictions.

9. The clinical decision support system according to one of the preceding claims, designed to output information about which input group of parameters affect the output the most, preferably designed to generate for individual parameters of this group the value of how much they affect the output result (R).

10. A prediction-method of computed decision support comprising the steps:
- providing a clinical decision support system (1) according to any of the preceding claims,
- providing input data (D) to the clinical decision support system (1), wherein the input data (D) is preferably selected and provided automatically, especially if new data becomes available for a predefined patient,
- determining a result (R) with the clinical decision support system (1) wherein a prediction model (M) is selected automatically by the clinical decision support system (1) based on the input data (D) and the result (R) is determined automatically by the selected prediction model (M),
- outputting the result (R), especially wherein a user is notified if substantial changes in a result for a patient occurred compared to earlier results for the same patient, e.g., in the form of a warning message or an icon in the patient list, so that he knows that he has to open the case.

11. A method for manufacturing a clinical decision support system (1) according to any of claims 1 to 9, comprising the steps:
- providing at least a first model-group (G) and a second model-group (G), each model-group (G) having a plurality of untrained machine learning models (m), especially with a number of models (m) having a different internal architecture and/or different hyperparameters,
- providing at least a first training-dataset (T) and a second training-dataset (T), each training-dataset (T) comprising data with a different distinguishing feature,
- training of the first model-group (G) with the first training-dataset (T) and the second model-group (G) with the second training-dataset (T),
- ranking each trained prediction model (M) of a model-group (G) with predefined quality-criteria, preferably wherein prediction models (M) are developed and compared offline,
- choosing the best ranked prediction model (M) of each model-group (G) as prediction model (M) for the clinical decision support system (1) manually or automatically.

12. The method according to claim 11, wherein a prediction method according to claim 10 is performed with the clinical decision support system (1) and a feedback-dataset is provided for a number of patients, wherein the prediction models (M) are further trained with this feedback dataset, the prediction models (M) being connected to the distinguishing feature of the feedback data, wherein a feedback-dataset is preferably used for training, where a patient had a flare with a DAS28-ESR score higher than 2.6.

13. A data processing system (7), especially a computer network system, comprising a data-network (N), a number of client computers (8) and a service computer-system (9), wherein the service computer system (9) comprises a clinical decision support system (1) according to one of claims 1 to 9.

14. A computer program product comprising a computer program that is directly loaded into a memory of a control unit of a computer system and which comprises program elements for performing steps of the method according to any of claims 10 to 12 when the computer program is executed by the control unit of the computer system.

15. A computer-readable medium on which is stored program elements that can be read and executed by a computer unit in order to perform steps of the method according to any of claims 10 to 12 when the program elements are executed by the computer unit.

## Patentansprüche

1. Klinisches Entscheidungsunterstützungssystem (1) zur Abschätzung der medikamentösen Behandlungsoptimierung im Zusammenhang mit entzündlichen Erkrankungen und dazu ausgebildet, eine Wahrscheinlichkeit eines Schubs im Zusammenhang mit der Verabreichung und/oder einer Dosierung eines vordefinierten Medikaments auszugeben, wobei das klinische Entscheidungsunterstützungssystem (1) umfasst:
- eine Recheneinheit (2), die dazu ausgebildet ist, mehrere Vorhersagemodelle (M) zu hosten, wobei die Recheneinheit (2) eine Eingabeschnittstelle (3), die dazu ausgebildet ist, Eingabedaten (D) zu empfangen, und eine Ausgabeschnittstelle (4), die dazu ausgebildet ist, Ergebnisse (R) auszugeben, umfasst,
- mehrere verschiedene trainierte Vorhersagemodelle (M), wobei jedes Modell (M) darauf trainiert wird, die Wahrscheinlichkeit von Behandlungsergebnissen für eine Anzahl verschiedener Optionen der medikamentösen Behandlung und für eine spezifische Patientengruppe basierend auf Eingabedaten vorherzusagen, und wobei eine Anzahl der verschiedenen Vorhersagemodelle darauf trainiert wird, ein Risiko von Schüben für verschiedene Szenarien einer Reduzierung der Medikamentendosis zu bestimmen,
- eine Auswahleinheit (6), die dazu ausgebildet ist, automatisch eines dieser Vorhersagemodelle (M) in Abhängigkeit von den Eingangsdaten (D) gemäß einem vordefinierten Auswahlschema auszuwählen,
wobei das klinische Entscheidungsunterstützungssystem (1) dazu ausgebildet ist, Ausgangsergebnisse (R) durch Verarbeiten der Eingangsdaten (D) mit dem ausgewählten Vorhersagemodell (M) zu erzeugen,
und wobei die Auswahleinheit dazu ausgebildet ist, die Eingabedaten nach vordefinierten Datentypen und/oder nach Werten von vordefinierten Datentypen zu durchsuchen und zu bestimmen, ob ein vordefinierter Datentyp in den Eingabedaten vorhanden ist und/oder einen Wert mit einem vordefinierten Schwellenwert zu vergleichen und/oder zu entscheiden, ob der Wert eine vordefinierte Anforderung erfüllt.

2. Klinisches Entscheidungsunterstützungssystem nach Anspruch 1, wobei für eine Anzahl der verschiedenen Vorhersagemodelle (M) jedes Vorhersagemodell (M) für eine andere Patientengruppe trainiert wurde und basierend auf patientenbezogenen Informationen in den Eingabedaten (D) ausgewählt wird, vorzugsweise basierend auf demographischen Daten und/oder auf Untersuchungsdaten, insbesondere basierend auf Informationen bezüglich Unterscheidungsmerkmalen aus der Gruppe, die Geschlecht, Art der Erkrankung (z.B. seropositive vergl. mit seronegative rheumatoide Arthritis), Alter, zugrunde liegender Gesundheitszustand, Body-Mass-Index umfasst.

3. Klinisches Entscheidungsunterstützungssystem nach einem der vorstehenden Ansprüche, wobei für eine Anzahl der unterschiedlichen Vorhersagemodelle (M) jedes Vorhersagemodell (M) für eine andere Stelle in einem klinischen Verlauf trainiert worden ist und basierend auf Eingabedaten (D) ausgewählt wird, die sich auf die Stelle des Patienten in einem klinischen Verlauf beziehen, vorzugsweise basierend auf Untersuchungsdaten.

4. Klinisches Entscheidungsunterstützungssystem nach einem der vorstehenden Ansprüche, wobei für eine Anzahl der verschiedenen Vorhersagemodelle (M) jedes Vorhersagemodell (M) für eine andere Medikation trainiert worden ist und basierend auf einer Art von Medikation, die in den Eingabedaten (D) angegeben ist, ausgewählt wird, wobei die Medikation insbesondere auf DMARDs, z.B. bDMARDs, cDMARDs oder tsDMARDs, oder NSAIDs basiert.

5. Klinisches Entscheidungsunterstützungssystem nach einem der vorstehenden Ansprüche, das dazu ausgebildet ist, ein Vorhersagemodell (M) basierend auf den Arten der verfügbaren Eingabedaten (D) auszuwählen, wobei vorzugsweise ein Vorhersagemodell (M) in Abhängigkeit davon ausgewählt wird, ob Labordaten Teil der Eingabedaten (D) sind oder nicht.

6. Klinisches Entscheidungsunterstützungssystem nach einem der vorstehenden Ansprüche, wobei eine Anzahl der verschiedenen Vorhersagemodelle (M) darauf trainiert wird, eine Wahrscheinlichkeit, dass ein einzelner Patient auf ein bestimmtes Medikament ansprechen wird, und/oder ein Risiko von Schüben für verschiedene Szenarien einer Reduzierung der Medikamentendosis zu bestimmen, wobei vorzugsweise ein Vorhersagemodell (M) trainiert wird zum
- Bestimmen einer Ansprechwahrscheinlichkeit für Medikamente der ersten Wahl, z. B. Methotrexat und Sulfasalazin, und/oder
- Bestimmen einer Auswahl des Medikaments der zweiten Wahl und/oder
- ein Szenario einer Reduzierung der Medikamentendosis in einer späteren Behandlungsphase, insbesondere für RA-Patienten, die Biologika erhalten, in stabiler Remission, vorzugsweise für mehrere Dosierungsschemata.

7. Klinisches Entscheidungsunterstützungssystem nach einem der vorstehenden Ansprüche, wobei eine Anzahl von Vorhersagemodellen (M) trainiert wird, um das Ansprechen eines Patienten auf ein Medikament für mehrere Medikamente zu bestimmen, wobei vorzugsweise ein einziges Modell das Ansprechen eines Patienten auf ein Medikament für mehrere Medikamente bestimmt und/oder ein Modell einer Gruppe von mehreren Modellen das Ansprechen eines Patienten auf ein Medikament für ein einziges Medikament bestimmt.

8. Klinisches Entscheidungsunterstützungssystem nach einem der vorstehenden Ansprüche, das dazu ausgebildet ist, eine Wahrscheinlichkeit eines Schubes, insbesondere im Zusammenhang mit der Verabreichung und/oder einer Dosierung eines vordefinierten Medikaments, eine Wahrscheinlichkeit eines unerwünschten Ereignisses (z.B. Nebenwirkungen von Medikamenten) und/oder eine Wahrscheinlichkeit des Nichtansprechens eines Patienten auf ein Medikament auszugeben,
wobei vorzugsweise Vorhersagemodelle (M) des klinischen Entscheidungsunterstützungssystems (1) darauf trainiert werden, einen Konfidenzwert für eine Vorhersage zu bestimmen und auszugeben, wobei vorzugsweise eine Vorhersage ein binärer Wert ist, der sich auf eine Klassifizierung bezieht, und die Konfidenz ein Wahrscheinlichkeitswert ist und/oder wobei vorzugsweise die Vorhersage eine Regression ist, die Ausgabe Vorhersageintervalle für punktuelle Vorhersagen umfasst.

9. Klinisches Entscheidungsunterstützungssystem nach einem der vorstehenden Ansprüche, das dazu ausgebildet ist, Informationen darüber ausgeben, welche Gruppe von Eingabeparametern die Ausgabe am stärksten beeinflusst, vorzugsweise dazu ausgebildet, für einzelne Parameter dieser Gruppe den Wert zu generieren, wie stark sie das Ausgabeergebnis (R) beeinflussen.

10. Vorhersage-Verfahren zur rechnerischen Entscheidungsunterstützung, die Schritte umfassend:
- Bereitstellen eines klinischen Entscheidungsunterstützungssystems (1) nach einem der vorstehenden Ansprüche,
- Bereitstellen von Eingabedaten (D) an das klinische Entscheidungsunterstützungssystem (1), wobei die Eingabedaten (D) vorzugsweise automatisch ausgewählt und bereitgestellt werden, insbesondere wenn neue Daten für einen vordefinierten Patienten verfügbar werden,
- Bestimmen eines Ergebnisses (R) mit dem klinischen Entscheidungsunterstützungssystem (1), wobei ein Vorhersagemodell (M) automatisch durch das klinische Entscheidungsunterstützungssystem (1) ausgewählt wird, basierend auf den Eingabedaten (D), und das Ergebnis (R) automatisch durch das ausgewählte Vorhersagemodell (M) bestimmt wird,
- Ausgeben des Ergebnisses (R), wobei insbesondere ein Benutzer benachrichtigt wird, wenn wesentliche Änderungen eines Ergebnisses für einen Patienten im Vergleich zu früheren Ergebnissen für denselben Patienten aufgetreten sind, z. B. in Form einer Warnmeldung oder eines Symbols in der Patientenliste, damit er weiß, dass er den Fall öffnen muss.

11. Verfahren zum Herstellen eines klinischen Entscheidungsunterstützungssystems (1) nach einem der Ansprüche 1 bis 9, die Schritte umfassend:
- Bereitstellen wenigstens einer ersten Modellgruppe (G) und einer zweiten Modellgruppe (G), wobei jede Modellgruppe (G) mehrere untrainierte Maschinenlernmodelle (m) aufweist, insbesondere mit einer Anzahl von Modellen (m), die eine unterschiedliche interne Architektur und/oder unterschiedliche Hyperparameter aufweisen,
- Bereitstellen wenigstens eines ersten Trainingsdatensatzes (T) und eines zweiten Trainingsdatensatzes (T), wobei jeder Trainingsdatensatz (T) Daten mit einem anderen Unterscheidungsmerkmal umfasst,
- Trainieren der ersten Modellgruppe (G) mit dem ersten Trainingsdatensatz (T) und der zweiten Modellgruppe (G) mit dem zweiten Trainingsdatensatz (T),
- Einstufen jedes trainierten Vorhersagemodells (M) einer Modellgruppe (G) anhand vordefinierter Qualitätskriterien, wobei vorzugsweise die Vorhersagemodelle (M) offline entwickelt und verglichen werden,
- Auswählen des bestplatzierten Vorhersagemodells (M) jeder Modellgruppe (G) als Vorhersagemodell (M) für das klinische Entscheidungsunterstützungssystem (1) manuell oder automatisch.

12. Verfahren nach Anspruch 11, wobei ein Vorhersageverfahren nach Anspruch 10 mit dem klinischen Entscheidungsunterstützungssystem (1) durchgeführt wird und ein Rückmeldungsdatensatz für eine Anzahl von Patienten bereitgestellt wird, wobei die Vorhersagemodelle (M) ferner mit diesem Rückmeldungsdatensatz trainiert werden, wobei die Vorhersagemodelle (M) mit dem Unterscheidungsmerkmal der Rückmeldungsdaten verbunden sind, wobei vorzugsweise ein Rückmeldungsdatensatz zum Trainieren verwendet wird, bei dem ein Patient einen Schub mit einem DAS28-ESR-Wert von mehr als 2,6 hatte.

13. Datenverarbeitungssystem (7), insbesondere Computernetzsystem, umfassend ein Datennetz (N), eine Anzahl von Client-Computern (8) und ein Dienstcomputersystem (9), wobei das Dienstcomputersystem (9) ein klinisches Entscheidungsunterstützungssystem (1) nach einem der Ansprüche 1 bis 9 umfasst.

14. Computerprogrammprodukt, umfassend ein Computerprogramm, das direkt in einen Speicher einer Steuereinheit eines Computersystems geladen wird und das Programmelemente zum Durchführen von Schritten des Verfahrens nach einem der Ansprüche 10 bis 12 umfasst, wenn das Computerprogramm durch die Steuereinheit des Computersystems ausgeführt wird.

15. Computerlesbares Medium, auf dem Programmelemente gespeichert sind, die durch eine Computereinheit gelesen und ausgeführt werden können, um Schritte des Verfahrens nach einem der Ansprüche 10 bis 12 auszuführen, wenn die Programmelemente durch die Computereinheit ausgeführt werden.

## Revendications

1. Un système (1) d'assistance à la décision clinique pour estimer une optimisation d'un traitement, lié à un médicament, concernant des maladies inflammatoires et conçu pour sortir une probabilité d'une poussée reliée à l'application et/ou à un dosage d'une médication définie à l'avance, le système (1) d'assistance à la décision clinique, comprenant :
- une unité (2) informatique conçue pour loger une pluralité de modèles (M) de prévision, l'unité (2) informatique comprenant une interface (3) d'entrée conçue pour recevoir une donnée (D) d'entrée et une interface (4) de sortie conçue pour sortir des résultats (R),
- une pluralité de différents modèles (M) de prévision d'apprentissage, dans lequel chaque modèle (M) subit un apprentissage pour prévoir la probabilité d'évolutions du traitement pour un nombre d'options de traitement différentes liées à un médicament et pour un groupe précis de patient sur la base d'une donnée d'entrée, et dans lequel un nombre des modèles de prévision différents subit un apprentissage pour déterminer le danger de poussées pour des scénarii différents de diminution progressive du médicament,
- une unité (6) de sélection conçue pour sélectionner automatiquement l'un des ces modèles (M) de prévision en fonction de la donnée (D) d'entrée suivant un plan de sélection défini à l'avance,
dans lequel le système (1) d'assistance à la décision clinique est conçu pour produire des résultats (R) de sortie en traitant la donnée (D) d'entrée par le modèle (M) de prévision sélectionné,
et dans lequel l'unité de sélection est conçue pour chercher la donnée d'entrée pour des types de données définis à l'avance et/ou pour des valeurs de types de données définis à l'avance et pour déterminer si un type de données défini à l'avance est présent dans la donnée d'entrée et/ou pour comparer une valeur à un seuil défini à l'avance et/ou pour décider si la valeur s'adapte à une exigence définie à l'avance.

2. Le système d'assistance à la décision clinique suivant la revendication 1, dans lequel, pour un nombre des modèles (M) de prévision différents, chaque modèle (M) de prévision a subi un apprentissage pour un groupe de patients différent et est sélectionné sur la base de l'information se rapportant au patient dans la donnée (D) d'entrée, de préférence sur la base de données démographiques et/ou de données d'examen, en particulier sur la base d'une information concernant des caractéristiques distinctives dans le groupe comprenant le genre, le type de maladie (par exemple la polyarthrite rhumatoïde séropositive vs séronégative), l'âge, l'état de santé sous-jacent, l'indice de masse corporelle.

3. Le système d'assistance à la décision clinique suivant l'une des revendications précédentes, dans lequel, pour un nombre de modèles (M) de prévision différents, chaque modèle (M) de prévision a subi un apprentissage pour un emplacement différent dans un chemin clinique et est sélectionné sur la base d'une donnée (D) d'entrée se référant à l'emplacement du patient dans un chemin clinique, de préférence sur la base de données d'examen.

4. Le système d'assistance à la décision clinique suivant l'une des revendications précédentes, dans lequel, pour un nombre des modèles (M) de prévision différents, chaque modèle (M) de prévision a subi un apprentissage pour une médication différente et est sélectionné sur la base d'un type de médication donné dans la donnée (D) d'entrée, la médication reposant en particulier sur des DMARDs, par exemple des bDMARDs, des cDMARDs ou des tsDMARDs, ou sur NSAIDs.

5. Le système d'assistance à la décision clinique suivant l'une des revendications précédentes, conçu pour sélectionner un modèle (M) de prévision sur la base des types de données (D) d'entrée disponibles, dans lequel, de préférence un modèle (M) de prévision est sélectionné en fonction du point de savoir si ou non une donnée de laboratoire fait partie de la donnée (D) d'entrée.

6. Le système d'assistance à la décision clinique suivant l'une des revendications précédentes, dans lequel un nombre de modèles (M) de prévision différents subit un apprentissage pour déterminer une probabilité qu'un patient individuel réagira à un médicament précis et/ou à un danger de poussées pour des scénarii différents de diminution progressive du médicament, dans lequel, de préférence un modèle (M) de prévision subit un apprentissage pour
- déterminer une probabilité de réaction pour des médicaments de première ligne, par exemple du méthotrexate et de la sulfasalazine, et/ou
- déterminer une sélection du médicament de deuxième ligne, et/ou
- un scénario de diminution progressive du médicament dans un stade ultérieur du traitement, en particulier pour des patients RA recevant des produits biologiques en rémission stable, de préférence pour une pluralité de régimes de dose.

7. Le système d'assistance à la décision clinique suivant l'une des revendications précédentes, dans lequel un nombre de modèles (M) de prévision subit un apprentissage pour déterminer une réaction au médicament d'un patient pour une pluralité de médicaments, dans lequel, de préférence un seul modèle détermine une réaction au médicament d'un patient pour une pluralité de médicaments et/ou un modèle parmi un groupe de multiples modèles détermine la réaction au médicament d'un patient pour un seul médicament.

8. Le système d'assistance à la décision clinique suivant l'une des revendications précédentes, conçu pour sortir une probabilité d'une poussée, reliée en particulier à l'application et/ou à un dosage d'une médication défini à l'avance, une probabilité d'un évènement néfaste (par exemple des effets secondaires d'une médication) et/ou une probabilité qu'un patient ne réagisse pas à un médicament,
dans lequel, de préférence des modèles (M) de prévision du système (1) d'assistance à la décision clinique subissent un apprentissage pour déterminer et sortir un score de confiance pour une prévision, dans lequel de préférence une prévision est une valeur binaire se référant à une classification et la confiance est une valeur de probabilité et/ou dans lequel de préférence la prévision est une régression, la sortie comprenant des intervalles de prévision pour des prévisions de points.

9. Le système d'assistance à la décision clinique suivant l'une des revendications précédentes, conçu pour sortir de l'information sur le groupe d'entrées de paramètres, qui affecte le plus la sortie, conçu, de préférence, pour créer, pour des paramètres individuels de ce groupe, la valeur de combien ils affectent le résultat (R) de sortie.

10. Un procédé de prévision d'une assistance à la décision assistée par ordinateur comprenant les stades :
- on se procure un système (1) d'assistance à la décision clinique suivant l'une quelconque des revendications précédentes,
- on donne une donnée (D) d'entrée au système (1) d'assistance à la décision clinique, dans lequel la donnée (D) d'entrée est choisie de préférence et donnée automatiquement, en particulier si la donnée nouvelle devient disponible pour un patient défini à l'avance,
- on détermine un résultat (R) par le système (1) d'assistance à la décision clinique, dans lequel un modèle (M) de prévision est sélectionné automatiquement par le système (1) d'assistance à la décision clinique sur la base de la donnée (D) d'entrée et le résultat (R) est déterminé automatiquement par le modèle (M) de prévision sélectionné,
- on sort le résultat (R), dans lequel en particulier un utilisateur reçoit une notification si des changements substantiels du résultat pour un patient se sont produits par rapport à des résultats précédents pour le même patient, par exemple sous la forme d'un message d'alerte ou d'un icône dans la liste des patients, de sorte qu'il sait qu'il doit ouvrir le cas.

11. Un procédé de fabrication d'un système (1) d'assistance à la décision clinique suivant l'une quelconque des revendications 1 à 9, comprenant les stades :
- on se procure au moins un premier groupe (G) de modèles et un deuxième groupe (G) de modèles, chaque groupe (G) de modèles ayant une pluralité de modèles (m) d'enseignement automatique n'ayant pas subi un apprentissage ayant, en particulier un nombre de modèles (m) ayant une architecture interne différente et/ou des hyperparamètres différents,
- on se procure au moins un premier ensemble (T) de données d'apprentissage et un deuxième ensemble (T) de données d'apprentissage, chaque ensemble (T) de données d'apprentissage comprenant une donnée ayant une caractéristique distinctive différente,
- on fait subir un apprentissage au premier groupe (G) de modèles par le premier ensemble (T) de données d'apprentissage et au deuxième groupe (G) de modèles par le deuxième ensemble (T) de données d'apprentissage,
- on classe chaque modèle (M) de prévision ayant subi un apprentissage d'un groupe (G) de modèles par un critère de qualité défini à l'avance, dans lequel, de préférence on développe des modèles (M) de prévision et on les compare hors ligne,
- on choisit manuellement ou automatiquement le modèle (M) de prévision ayant le meilleur classement de chaque groupe (G) de modèles comme modèle (M) de prévision pour le système (1) d'assistance à la décision clinique.

12. Le procédé suivant la revendication 11, dans lequel on effectue un procédé de prévision suivant la revendication 10 avec le système (1) d'assistance à la décision clinique et on se procure un ensemble de données de réaction pour un nombre de patients, dans lequel les modèles (M) de prévision subissent en outre un apprentissage avec cet ensemble de données de réaction, les modèles (M) de prévision étant reliés à la caractéristique distinctive de la donnée de réaction, dans lequel un ensemble de données de réaction est utilisé de préférence pour l'apprentissage, lorsqu'un patient a une rechute ayant un score DAS28-ESR supérieur à 2,6.

13. Un système (7) de traitement de données, en particulier système à réseau d'ordinateurs, comprenant un réseau (N) de données, un nombre d'ordinateurs (8) clients et un système (9) informatique de service, dans lequel le système (9) informatique de service comprend un système (1) d'assistance à la décision clinique suivant l'une des revendications 1 à 9.

14. Un produit de programme d'ordinateur comprenant un programme d'ordinateur, qui est chargé directement dans la mémoire d'une unité de commande d'un système informatique, qui comprend des éléments de programme pour effectuer des stades du procédé suivant l'une quelconque des revendications 10 à 12, lorsque le programme d'ordinateur est exécuté par une unité de commande du système informatique.

15. Un support, déchiffrable par ordinateur, sur lequel sont mis en mémoire des éléments de programme, qui peuvent être lus et exécutés par une unité informatique afin d'effectuer des stades du procédé suivant l'une quelconque des revendications 10 à 12.
